# DEMANDE DE BREVET EUROPEEN

(11) **EP 0 736 251 A1**
(43) Date de publication de la demande: **09.10.1996**
(21) Numéro de dépôt: 96400758.7
(22) Date de dépôt: 05.04.1996
(51) Int. Cl.: A01N 37/02, A61K 7/06

(54) **Lotion antiparasitaire**

(30) Priorité: 07.04.1995 FR 9504197
(71) Demandeur: Benwaiche, Joseph, 64200 Arcangues (FR)
(72) Inventeur: Benwaiche, Joseph, 64200 Arcangues (FR)
(74) Mandataire: Sabatier, Marc

(57) **Abrégé**

Lotion antiparasitaire aqueuse ou alcoolisée, caractérisée en ce qu'elle renferme les composants suivants :
- Acide acétique;
- Hexachlorocyclohexane officinal (HCH)
- Acétone;
- Solvant.

## Description

La présente invention concerne une lotion antiparasitaire plus particulièrement destinée au traitement des insectes et surtout des acariens tels que poux ou puces dans les cheveux ou poils mais également aoûtats ou tiques en ce qui concerne les animaux. A l'heure actuelle, il existe sur le marché un certain nombre de produits de ce type dont les principaux sont la pyrithione et la malathion.

La pyrithione est en fait une désignation abrégée pour le pyridinethiol -1- oxyde. C'est en fait un fongicide et un bactéricide contre les pellicules et les insectes.

La malathion est quand à elle une appellation commune pour insecticides et acaricides S -(1,2 - Dicarbethoxythyl) -0,0,-Bimethyl dithiophosphato utilisée contre les insectes résistant au DDT.

Il s'agit là en fait de composants entrant dans la composition de lotions antiparasitaires plus connues sous les appellations commerciales de PRIODERM ou MARIE-ROSE, citées exclusivement à titre d'exemple illustratifs de l'art antérieur. Ces produits donnent des résultats généralement satisfaisants, mais il a pu être constaté que lors d'applications répétées pour un traitement intensif de longue durée; dans des cas difficiles, les insectes ou acariens parasites présentent un phénomène d'accoutumance à ces produits et deviennent plus résistants, d'où une plus grande difficulté à les exterminer.

En ce qui concerne le PRIODERM, sa composition est la suivante :
- MALATION 0,50 g pour 100 ml ou 0,55 g pour 110 ml
- Excipients : Terpinol, Dipentène parfum (pin), isopropanol

En ce qui concerne la MARTE-ROSE pour un flacon de 125 ml, sa composition est la suivante :
- Pyrethrines naturelles 0,30 g;
- Acide acétique 4,00 g;
- Excipient parfumé QSP 100 ml.

L'invention propose une lotion antiparasitaire au moins aussi efficace que celles de l'art antérieur, mais qui de plus évite les phénomènes d'accoutumance précités, mettant en oeuvre des composants nouveaux au sens qu'ils n'avaient à ce jour, encore jamais été introduits dans ce type de lotion.

L'invention concerne une lotion du type précité, aqueuse ou alcoolisée, caractérisée en ce qu'elle renferme les composants suivants :
- Acide acétique
- Hexachlorocyclohexane officinal (HCH)
- Acétone
- Solvant.

Dans la version aqueuse les composants sont utilisés dans les proportions suivantes :
- Acide acétique 37,5 à 62,5 g
- Hexachlorocyclohexane officinal 7,5 à 12,5 g
- Acétone 75,0 à 125,0 g
- Eau en quantité suffisante pour 1,5 l de solution

Dans la version alcoolisée de la lotion, le solvant est de l'alcool à 70° et comprend en outre du formol. Ceci a pour avantage de stabiliser la solution et de lui assurer une meilleure conservation.

Selon cette même version alcoolisée la solution est rendue d'un usage agréable en y incorporant un alcoolat de lavande.

En fait, selon cette deuxième variante, la lotion selon l'invention comprend les composants précités dans les proportions suivantes :
- Acide acétique 37,5 à 62,5 g
- Hexachlorocyclohexane officinal (HCH) 7,5 à 12,5 g
- Acétone 75,0 à 125,0 g
- Formol 3,75 à 6,25 g
- Alcool de lavande 2,25 à 3,75 g
- Alcool à 70° en quantité suffisante pour 1,5 l de solution

La solution alcoolisée présente un avantage supplémentaire par rapport à la solution aqueuse en ce sens qu'elle sèche plus rapidement sur les cheveux et donc est plus pratique d'utilisation tout en restant très performante.

Selon des proportions préférentielles des composants, une solution type consiste dans la formulation suivante :
- Acide acétique 50 g
- Hexachlorocyclohexane officinal (HCH) 10 g
- Acétone 100 g
- Formol 5 g
- Alcool de lavande 3 g
- Alcool à 70° en quantité suffisante pour 1,5 l de solution

## Revendications

1. Lotion antiparasitaire aqueuse ou alcoolisée, caractérisée en ce qu'elle renferme les composants suivants :
- Acide acétique;
- Hexachlorocyclohexane officinal (HCH) ;
- Acétone;
- Solvant.

2. Lotion selon la revendication 1, caractérisée en ce que le solvant est de l'eau et en ce que les composants sont utilisés dans les proportions suivantes :
- Acide acétique 37,5 à 62,5 g
- Hexachlorocyclohexane officinal (HCH) ; 7,5 à 12,5 g
- Acétone 75,0 à 125,0 g
- Eau en qantité suffisante pour 1,5 l de solution

3. Lotion selon la revendication 1, caractérisée en ce que le solvant est de l'alcool.

4. Lotion selon la revendication 1, caractérisée en ce qu'elle comprend en outre un alcoolat de lavande.

5. Lotion selon les revendications 3 ou 4, caractérisée en ce qu'elle comprend en outre du formol.

6. Lotion selon les revendications 3 à 5, caractérisée en ce que ses composants sont utilisés dans les proportions suivantes :
- Acide acétique 37,5 à 62,5 g
- Hexachlorocyclohexane officinal (HCH) 7,5 à 12,5 g
- Acétone 75,0 à 125,0 g
- Formol 3,75 à 6,25 g
- Alcoolat de lavande 2,25 à 3,75 g
- Alcool 70° en quantité suffisante pour 1,5 l de solution
